(19) Europäisches Patentamt
European Patent Office
Office européen des brevets

(11) **EP 3 758 610 B1**

(12) **FASCICULE DE BREVET EUROPEEN**

(45) Date de publication et mention de la délivrance du brevet:
**03.04.2024   Bulletin 2024/14**

(21) Numéro de dépôt: **19706655.8**

(22) Date de dépôt: **26.02.2019**

(51) Classification Internationale des Brevets (IPC):
**A61B 8/08** (2006.01)   **A61B 8/00** (2006.01)
**G01S 7/52** (2006.01)

(52) Classification Coopérative des Brevets (CPC):
**A61B 8/485; A61B 8/08; A61B 8/4444; A61B 8/54; G01S 7/52042; G01S 7/52071; G01S 7/52073; G01S 7/52079**

(86) Numéro de dépôt international:
**PCT/EP2019/054656**

(87) Numéro de publication internationale:
**WO 2019/166393 (06.09.2019 Gazette 2019/36)**

(54) **PROCÉDÉ DE MESURE D'UN PARAMÈTRE D'ATTENUATION ULTRASONORE GUIDE PAR ÉLASTOGRAPHIE HARMONIQUE, SONDE ET DISPOSITIF POUR LA MISE EN OEUVRE DU PROCÉDÉ**

VERFAHREN ZUR MESSUNG EINES ULTRASCHALLDÄMPFUNGSPARAMETERS, GEFÜHRT DURCH HARMONISCHE ELASTOGRAFIE, SONDE UND VORRICHTUNG ZUR DURCHFÜHRUNG DES BESAGTEN VERFAHRENS

METHOD FOR MEASURING AN ULTRASONIC ATTENUATION PARAMETER, GUIDED BY HARMONIC ELASTOGRAPHY; PROBE; AND DEVICE FOR IMPLEMENTING SAID METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **02.03.2018   FR 1851822**

(43) Date de publication de la demande:
**06.01.2021   Bulletin 2021/01**

(73) Titulaire: **Echosens**
**75014 Paris (FR)**

(72) Inventeurs:
• **SANDRIN, Laurent**
**92340 Bourg-La-Reine (FR)**
• **AUDIERE, Stéphane**
**75012 Paris (FR)**

(74) Mandataire: **Cabinet Camus Lebkiri**
**25 rue de Maubeuge**
**75009 Paris (FR)**

(56) Documents cités:
**WO-A1-2016/069750**

• **MELLEMA DANIEL C ET AL: "Probe Oscillation Shear Elastography (PROSE): A High Frame-Rate Method for Two-Dimensional Ultrasound Shear Wave Elastography", IEEE TRANSACTIONS ON MEDICAL IMAGING, IEEE SERVICE CENTER, PISCATAWAY, NJ, US, vol. 35, no. 9, 1 septembre 2016 (2016-09-01), pages 2098-2106, XP011621555, ISSN: 0278-0062, DOI: 10.1109/TMI.2016.2550007 [extrait le 2016-09-01]**
• **Sandrin Laurent ET AL: "Chapter 19: Non-Invasive Assessment of Liver Fibrosis by Vibration-Controlled Transient Elastography (Fibroscan?)" In: "Liver Biopsy", 6 septembre 2011 (2011-09-06), XP055411434, ISBN: 978-953-30-7644-7 section "2.3. Background of quantitative elastography" section "3. Fibroscan device"**

## Description

## DOMAINE TECHNIQUE

L'invention appartient au domaine de la mesure de l'atténuation ultrasonore et plus particulièrement de l'utilisation de l'élastographie harmonique pour guider la détermination de l'atténuation ultrasonore d'un milieu présentant un signal ultrasonore après illumination ultrasonore. Un premier objet de l'invention est un procédé de mesure d'un paramètre d'atténuation ultrasonore guidé par élastographie harmonique. Un deuxième objet de l'invention est une sonde pour la mesure d'un paramètre d'atténuation ultrasonore guidée par élastographie harmonique. Un troisième objet de l'invention est un dispositif pour la mesure de l'atténuation ultrasonore guidée par élastographie harmonique. Le procédé de mesure de l'atténuation ultrasonore guidé par élastographie harmonique est particulièrement adapté pour déterminer les propriétés d'un milieu viscoélastique tel que le foie humain ou animal.

## ETAT DE L'ART

Il est connu que l'atténuation ultrasonore est corrélée à la quantité de gras contenue dans le foie. Elle peut donc être utilisée pour mesurer la quantité de stéatose dans le foie.

La demanderesse a développé et commercialisé un dispositif qui quantifie de manière non invasive l'atténuation ultrasonore appelée CAP. La technique utilisée est la mesure de l'atténuation ultrasonore guidée par élastographie transitoire à vibration contrôlée (ou Vibration Controlled Transient Elastography, « VCTE »). La technique VCTE est décrite dans le document « Transient Elastography : a new noninvasive method for assessment of hepatic fibrosis » de L. Sandrin et al., publié dans Ultrasound in Medecine and Biology, Vol. 29, pages 1705-1713, 2003. L'étude des signaux ultrasonores acquis pour la mesure de l'élasticité permet de remonter à l'atténuation ultrasonore du milieu comme il est expliqué dans les documents :

- "The controlled attenuation parameter (CAP): A novel tool for the non-invasive évaluation of steatosis using Fibroscan®" de M. Sasso et al., publié dans Clinical Research in Hepatology and Gastroenterology, 2011 ;
- "Controlled Atténuation Parameter (CAP): A Novel VCTE™ Guided Ultrasonic Atténuation Measurement for the Evaluation of Hepatic Steatosis: Preliminary Study and Validation in a Cohort of Patients with Chronic Liver Disease from Various Causes" de M. Sasso et al., publié dans Ultrasound in Medecine and Biology, 2010 ;
- "Liver steatosis assessed by controlled attenuation parameter (cap) measured with the xl probe of the fibroscan: a pilot study assessing diagnostic accuracy" de M. Sasso et al. publié dans Ultrasound in Medecine and Biology, 2015.

Le dispositif mettant en oeuvre cette technique, appelé Fibroscan ®, est capable de mesurer l'élasticité et l'atténuation ultrasonore du foie humain de façon rapide, non-invasive et reproductible. Dans le FibroScan, la mesure de l'atténuation ultrasonore (CAP) est validée par la mesure d'élasticité : lorsque la mesure de l'élasticité est valide, la valeur de CAP associée est considérée comme valide. Il ne s'agit donc pas d'un guidage a priori mais d'une validation a posteriori. Pour mesurer avec précision l'atténuation ultrasonore, le FibroScan valide la mesure de CAP à partir du résultat de l'élastographie impulsionnelle.

Dans un tel dispositif pour élastographie transitoire, l'onde de cisaillement impulsionnelle est générée par un vibreur placé au contact du milieu à caractériser. La propagation de l'onde de cisaillement est ensuite suivie à l'aide d'une série d'acquisitions ultrasonores réalisées par un transducteur ultrasonore avec un taux de répétition élevé. Chaque acquisition ultrasonore correspond à au moins une émission ultrasonore. Chaque émission ultrasonore peut être associée à la détection et l'enregistrement à la volée des échos générés par les particules réfléchissantes présentes dans le milieu étudié pour une gamme de profondeurs définie. Les signaux ultrasonores réfléchis sont traités par corrélation pour remonter aux mouvements du tissu engendrés par la propagation de l'onde de cisaillement en fonction du temps et de la position dans le milieu. L'étude de ces mouvements permet de remonter à la vitesse de propagation de l'onde de cisaillement à l'intérieur du milieu viscoélastique puis à l'élasticité.

La mesure du CAP (atténuation ultrasonore) par la technique VCTE présente plusieurs limitations.

Le premier inconvénient de la mesure du CAP (atténuation ultrasonore) par la technique VCTE est la difficulté de prédire que la sonde est effectivement placée en regard du foie. En effet il est nécessaire de réaliser une mesure valide par élastographie impulsionnelle pour obtenir une mesure de l'atténuation ultrasonore.

Le deuxième inconvénient de la mesure du CAP par la technique VCTE est le coût du dispositif qui nécessite l'utilisation d'une vibration impulsionnelle dont le coût est élevé.

Le troisième inconvénient de la mesure du CAP par la technique VCTE est la nécessité de réaliser environ une dizaine de mesures d'élasticité par élastographie impulsionnelle dont la durée individuelle est d'au moins 1 seconde y compris le temps de calcul ce qui conduit à une durée d'examen d'environ une minute.

Aujourd'hui il est possible d'utiliser les ultrasons pour guider le positionnement du vibreur pour l'élastographie transitoire. Par exemple, il est possible d'utiliser l'imagerie ultrasonore ou un outil de ciblage comme celui décrit dans la demande de brevet EP2739211 A1. Toutefois, ces solutions ne sont pas satisfaisantes car elles ne permettent pas de prédire directement que la sonde est bien positionnée en regard de l'organe étudié. Par ailleurs, ces techniques sont très opérateur dépendantes. Enfin les signaux ultrasonores peuvent se révéler peu spécifique du foie.

Il existe par ailleurs des techniques d'élastographie dites harmoniques. Ces techniques reposent sur l'application d'une vibration continue ayant une fréquence comprise entre 30 Hz et 100 Hz. Les ondes élastiques créées à l'intérieur du milieu sont des ondes quasi-stationnaires, superpositions d'ondes de cisaillement et d'ondes de compression qui ne sont pas aussi performantes que les techniques d'élastographie impulsionnelle pour la mesure précise des propriétés viscoélastiques mais qui utilisent néanmoins des ondes de cisaillement et peuvent en mesurer la propagation.

Enfin il est connu que les ondes de cisaillement basse fréquence se propagent bien dans le foie. Cette constatation est vraie pour des ondes de cisaillement impulsionnelles et harmoniques.

Par ailleurs, la technique d'élastographie harmonique peut être utilisée pour guider des procédés de traitement. Il s'agit par exemple de traiter des tumeurs localisées par la technique d'élastographie harmonique au moyen de procédés de type hyperthermie.

## PROBLEME TECHNIQUE

Aucune technique de mesure de l'atténuation ultrasonore n'assure un guidage optimal et en temps réel de la sonde ultrasonore utilisée par rapport au tissu à caractériser. Il en résulte que la mesure peut se révéler difficile à réaliser compte tenu de l'impossibilité d'affirmer que l'organe étudié est bien situé en regard de la sonde. Ceci n'est pas favorable à une mise en oeuvre avec des dispositifs de petite taille et simple utilisation.

## RESUME DE L'INVENTION

Pour résoudre au moins partiellement ces problèmes, la présente invention décrit une nouvelle technique de mesure d'un paramètre d'atténuation ultrasonore qui est guidée par élastographie harmonique.

Un objet de la présente invention est un procédé de mesure d'un paramètre d'atténuation ultrasonore guidé par élastographie harmonique comprenant une étape d'application, à l'aide d'un vibreur compris dans une sonde au contact d'un milieu viscoélastique, d'une vibration continue basse fréquence, la vibration continue basse fréquence générant une onde élastique à l'intérieur du milieu viscoélastique et de génération, pendant la propagation de l'onde élastique, à l'aide d'un transducteur ultrasonore au contact du milieu viscoélastique, d'une série d'acquisitions ultrasonores, ladite série d'acquisitions ultrasonores comprenant des groupes d'acquisitions ultrasonores, les groupes d'acquisitions ultrasonores étant générés avec un taux de répétition, chaque groupe d'acquisitions ultrasonores comprenant au moins une acquisition ;

le paramètre d'atténuation ultrasonore étant mesuré à partir des acquisitions ultrasonores.

Selon un mode de réalisation les acquisitions ultrasonores sont réalisées pendant l'application de la vibration continue basse fréquence.

On entend par mesure d'atténuation ultrasonore guidée par élastographie harmonique un procédé comprenant au moins une étape d'application d'une vibration continue et la mesure d'un paramètre reflétant l'atténuation ultrasonore. En d'autres termes, le procédé selon l'invention comprend à la fois une génération d'une vibration continue, ce qui est caractéristique d'une technique d'élastographie harmonique, et la mesure d'un paramètre reflétant l'atténuation ultrasonore.

On entend par atténuation ultrasonore tout paramètre qui reflète l'atténuation ultrasonore : Broadband Ultrasound Atténuation (BUA, en dB/cm/MHz), l'atténuation mesurée à une fréquence particulière (en dB/cm), le Controlled Atténuation Parameter (CAP), etc.

On entend par vibration continue basse fréquence la reproduction en continu d'un motif de forme d'onde. Ce motif peut par exemple être une sinusoïde parfaite ; on parle alors de vibration monochromatique. La vibration peut également être constituée par la reproduction d'un motif arbitraire. La vibration continue est interrompue pour arrêter le processus de mesure ou lorsque les conditions de mesure ne sont plus satisfaites. Les conditions de mesure pouvant être par exemple une condition sur la force de contact avec le milieu étudié. La fréquence centrale de la vibration continue basse fréquence est comprise entre 5 et 500 Hz.

On entend par onde élastique la superposition d'ondes de compression et d'ondes de cisaillement.

On entend par acquisition ultrasonore l'émission d'un tir ultrasonore. Ladite émission ultrasonore peut être associée à la détection et l'enregistrement à la volée des échos générés par les particules réfléchissantes présentes dans une gamme de profondeurs définie du milieu étudié.

La série d'acquisitions ultrasonores est donc formée par une répétition de groupes d'acquisitions. Un groupe d'acquisitions comprend au moins une acquisition ultrasonore. Les groupes d'acquisitions sont émis ou générés avec un premier taux de répétition. Le premier taux de répétition est également appelé taux de répétition inter-groupe. Le premier taux de répétition est typiquement compris entre 5 et 500 Hz.

Quand chaque groupe d'acquisitions est formé par au moins deux acquisitions ultrasonores, les acquisitions ultrasonores formant un même groupe sont émises ou générées avec un taux de répétition intra-groupe typiquement compris entre 500 Hz et 100 kHz.

Avantageusement, l'utilisation d'un taux de répétition faible pendant l'application de la vibration continue permet de mesurer les déplacements du tissu viscoélastique tout en limitant l'énergie acoustique envoyée dans le tissu même de manière à ne pas dépasser les limites de puissance acoustique pic et moyenne.

Le terme déplacement est considéré au sens large dans ce document. Il englobe tout paramètre de mouvement comme le déplacement, la vitesse, la déformation, le taux de déformation, la vitesse de déformation et toute transformation mathématique appliquée à ces paramètres.

Lors de l'application de la vibration continue, une onde élastique est générée à l'intérieur du milieu viscoélastique

La série d'acquisitions ultrasonores est utilisée pour étudier la propagation de l'onde élastique à l'intérieur du milieu viscoélastique. Il est possible de détecter les échos ou signaux ultrasonores réfléchis par le milieu viscoélastique et de calculer, à partir de ces signaux ultrasonores réfléchis, les déplacements du milieu viscoélastique causés par la propagation de l'onde élastique à l'intérieur du milieu viscoélastique engendrée par la vibration continue.

A titre d'exemple, il est possible de calculer les déplacements du milieu viscoélastique en en appliquant une technique de corrélation aux acquisitions ultrasonores composant un même groupe d'acquisitions de la série d'acquisitions ultrasonores.

Il est important de noter que la propagation de l'onde élastique évolue en fonction de la position de la sonde au contact du milieu étudié.

Il est alors possible de mesurer une propriété de la propagation de l'onde élastique à l'intérieur du milieu et de calculer en temps réel un indicateur de positionnement à partir des propriétés mesurées. Idéalement cet indicateur de positionnement temps réel est affiché en temps réel pour guider l'opérateur afin qu'il trouve la meilleure position pour la sonde. Des exemples de propriétés mesurées pour calculer l'indicateur de positionnement sont l'amplitude et la phase de l'onde élastique, mesurées en fonction de la profondeur dans le tissu à caractériser. Il est également possible de calculer la vitesse de phase de l'onde élastique.

Dans la suite de ce document « indicateur de positionnement temps réel » et indicateur de positionnement » se réfèrent au même indicateur de positionnement temps réel.

On entend par temps réel un indicateur dont l'affichage est rafraichi régulièrement pendant l'examen. En général, le taux de rafraichissement est d'environ 20 Hz mais peut aussi être de l'ordre de 1 Hz.

En d'autres termes, l'indicateur de positionnement donne une probabilité de la présence du milieu viscoélastique à caractériser en regard du transducteur ultrasonore.

Il est important de noter que la vibration continue est avant tout utilisée pour vérifier le positionnement de la sonde utilisée pour la mesure de l'atténuation ultrasonore. A titre d'exemple la vibration continue peut être utilisée pour vérifier la présence du parenchyme hépatique en face de la sonde. En d'autres termes, pendant l'étape d'application d'une vibration continue, la mesure indirecte des propriétés viscoélastiques du milieu est possible mais pas indispensable. Toutefois, une valeur d'élasticité peut effectivement être déduite à partir de la vitesse de phase de l'onde élastique. En effet, la mesure des déplacements engendrés dans le milieu viscoélastique par ladite propagation permet ensuite de remonter à la vitesse de propagation de l'onde élastique. Si on fait l'hypothèse que l'onde élastique est principalement une onde de cisaillement, on peut alors calculer l'élasticité du milieu en utilisant la formule $E=3\rho V_s^2$ où E est l'élasticité ou module d'Young, $\rho$ la densité et $V_s$ la vitesse de l'onde élastique.

Le procédé de guidage d'une mesure d'atténuation ultrasonore par élastographie harmonique selon l'invention permet donc de prédire si le positionnement de la sonde est favorable en utilisant une technique d'élastographie harmonique. Il s'agit d'un guidage. Les mesures de l'atténuation ultrasonore réalisées en cours du temps peuvent être stockées dans une mémoire. En particulier chaque mesure d'atténuation ultrasonore peut être associée à un coefficient de qualité. En général le coefficient de qualité sera un coefficient compris entre 0 et 1. La valeur 0 correspondant à une mauvaise qualité et la valeur 1 à une très bonne qualité de mesure.

Le calcul du coefficient de qualité peut être effectué à partir des propriétés de propagation de l'onde élastique. Il peut également inclure des caractéristiques des signaux ultrasonores, et de critères de qualité émanant du calcul de l'atténuation ultrasonore.

La mesure d'atténuation ultrasonore finale retenue peut être calculée à partir des mesures d'atténuation ultrasonore et des coefficients de qualité stockés en mémoire.

En d'autres termes, le guidage par élastographie harmonique permet d'associer à chaque mesure d'atténuation ultrasonore un coefficient de qualité et de guider le positionnement de la sonde au regard du tissu à caractériser en fournissant à l'opérateur un indicateur de positionnement prédictif de la présence de l'organe étudié.

A titre d'exemple, la mesure d'atténuation ultrasonore retenue est la moyenne des mesures d'atténuation ultrasonore stockées pondérées par leurs coefficients de qualité. Le nombre total de mesures d'atténuation ultrasonore peut typiquement être compris entre 1 et plusieurs milliers de mesures. Par exemple, si les mesures sont accumulées pendant 60 secondes à raison de 20 mesures par seconde, N sera égal à 1200.

Avantageusement, le procédé de guidage d'une mesure d'atténuation ultrasonore par élastographie harmonique selon l'invention permet de réaliser une mesure d'atténuation ultrasonore du tissu à caractériser de façon fiable et reproductible en assurant un positionnement optimal de la sonde de façon simple et précise grâce à une technique d'élastographie harmonique.

Avantageusement, le procédé de guidage d'une mesure d'atténuation ultrasonore par élastographie harmonique selon l'invention permet de réduire les coûts de fabrication par l'utilisation d'un système vibratoire plus simple qu'en élastographie impulsionnelle.

Avantageusement, le procédé de guidage d'une mesure d'atténuation ultrasonore par élastographie harmonique selon l'invention permet de fournir un indicateur de positionnement temps réel à l'opérateur.

Le procédé de guidage d'une mesure d'atténuation ultrasonore par élastographie harmonique selon l'invention peut également présenter une ou plusieurs des caractéristiques ci-dessous, considérées individuellement ou selon toutes les combinaisons techniquement possibles :

- le procédé selon l'invention comprend en outre une étape de détermination, à partir de la série d'acquisitions ultrasonores, d'au moins une propriété de la propagation de l'onde élastique à l'intérieur du milieu viscoélastique ;

- la propriété de la propagation de l'onde élastique à l'intérieur du milieu viscoélastique est utilisée pour calculer un indicateur de positionnement temps réel de la sonde par rapport au milieu viscoélastique à étudier ;

- le procédé selon l'invention comprend en outre une étape d'affichage en temps réel de l'indicateur de positionnement temps réel ;

- l'étape d'application d'une vibration continue basse fréquence est déclenchée uniquement si la force de contact entre le vibreur et le milieu viscoélastique est supérieure à un seuil inférieur prédéterminé ;

- l'étape d'application d'une vibration continue basse fréquence est déclenchée uniquement si la force de contact entre le vibreur et le milieu viscoélastique est supérieure à un seuil inférieur et inférieure à un seuil supérieur prédéterminés ;

- la série d'acquisitions ultrasonores est formée par une répétition de groupes comprenant au moins deux acquisitions ultrasonores ayant un taux de répétition intra-groupe compris entre 500 Hz et 10 kHz et un taux de répétition compris entre 10 Hz et 10 kHz ;

- le taux de répétition est plus faible que la fréquence de la vibration continue ;

- un paramètre de l'onde ultrasonore qui reflète l'atténuation ultrasonore est calculée et affichée ;

- le paramètre d'atténuation ultrasonore est un paramètre instantané et un coefficient de qualité associé à la mesure du paramètre d'atténuation ultrasonore instantané est calculé à partir d'une propriété de la propagation de l'onde élastique à l'intérieur du milieu viscoélastique et/ou des propriétés de l'onde ultrasonore ; on entend par paramètre instantané une mesure obtenue à chaque acquisition ultrasonore ou à partir d'une seule acquisition ultrasonore ;

- un paramètre d'atténuation ultrasonore moyen est calculé à partir d'une pluralité de paramètres d'atténuation ultrasonore instantanés et des coefficient de qualités associés à chaque paramètre d'atténuation ultrasonore instantané ;

- les paramètres d'atténuation ultrasonores, associées ou non à des coefficients de qualité calculés sur la base des propriétés de la propagation de l'onde élastique à l'intérieur du milieu viscoélastique, sont stockées dans une mémoire ;

- le seuil inférieur prédéterminé de force de contact pour l'application de la vibration continue est typiquement choisi égal à 1 N ;

- la fréquence de la vibration basse fréquence, cSWF, appliquée par le vibreur est comprise entre 5 et 500 Hz ;

- l'amplitude de la vibration basse fréquence appliquée par le vibreur est comprise entre 10 $\mu$m et 5 mm ;

- la série d'acquisitions ultrasonores est formée par une répétition de groupes comprenant au moins deux acquisitions ultrasonores ayant un taux de répétition intra-groupe compris entre 500 Hz et 10 kHz et un premier taux de répétition compris entre 10 Hz et 10 kHz ;

- le premier taux de répétition est plus faible que la fréquence de la vibration continue ;

- le paramètre d'atténuation ultrasonore est choisie parmi

  ∘ le BUA
  ∘ l'atténuation d'une fréquence ultrasonore particulière ;
  ∘ Paramètre d'atténuation ultrasonore contrôlée (CAP) ;

- la propriété de la propagation de l'onde élastique est délivrée à l'opérateur ;

- la propriété de la propagation de l'onde élastique est choisie parmi :

  ∘ l'amplitude de l'onde élastique ;
  ∘ la phase de l'onde élastique ;
  ∘ le module d'Young du milieu viscoélastique ;
  ∘ le module de cisaillement du milieu viscoélastique ;
  ∘ la vitesse de cisaillement du milieu viscoélastique.

Un autre objet de la présente invention est une sonde pour la mise en oeuvre du procédé d'élastographie hybride selon l'invention. La sonde selon l'invention comprend :

- Un vibreur configuré pour appliquer au milieu viscoélastique une vibration continue basse fréquence, la vibration continue basse fréquence générant une onde élastique à l'intérieur du milieu viscoélastique ;
- Un transducteur ultrasonore configuré pour émettre une série d'acquisitions ultrasonores, ladite série d'acquisitions ultrasonores comprenant des groupes d'acquisitions ultrasonores, les groupes d'acquisitions ultrasonores étant générés avec un taux de répétition, chaque groupe d'acquisitions ultrasonores comprenant au moins une acquisition ;
- Des moyens de calcul et d'affichage en temps réel d'un indicateur de positionnement de la sonde, ledit indicateur de positionnement étant calculé à partir d'une propriété de la propagation de l'onde élastique, ladite propriété de propagation de l'onde élastique étant déterminée à partir de la série d'acquisitions ultrasonores ;

le paramètre d'atténuation ultrasonore étant mesuré à partir des acquisitions ultrasonores réalisées pendant l'application de la vibration continue basse fréquence.

Selon un mode de réalisation, ladite sonde est en outre configurée appliquer la vibration continue lorsque la force de contact entre la sonde et le milieu viscoélastique est supérieure à une valeur prédéterminée.

La sonde selon l'invention permet la mise en oeuvre du procédé selon l'invention.

Le transducteur ultrasonore est utilisé pour envoyer la série d'acquisitions ultrasonores à l'intérieur du milieu viscoélastique. Le même transducteur ultrasonore détecte les signaux ultrasonores réfléchis à chaque acquisition. Les signaux ultrasonores réfléchis sont ensuite traités pour détecter les déplacements du milieu viscoélastique engendrés par les ondes élastiques.

On entend par moyens de calcul au moins un microprocesseur et une mémoire destinée à stocker les acquisitions ultrasonores et les résultats des calculs telle qu'un indicateur de positionnement de la sonde ou une propriété de la propagation de l'onde élastique.

On entend par moyen d'affichage un écran ou un indicateur configuré pour afficher l'indicateur de positionnement. L'indicateur peut être par exemple un indicateur lumineux tel qu'une diode ou un indicateur sonore.

La sonde pour élastographie hybride selon l'invention peut également présenter une ou plusieurs des caractéristiques ci-dessous, considérées individuellement ou selon toutes les combinaisons techniquement possibles :

- le vibreur est un moteur électrique ou une bobine audio ou un actionneur électrodynamique ;

- le transducteur ultrasonore est monté sur l'axe du vibreur ;

- la sonde selon l'invention comprend en outre des moyens pour déclencher l'accumulation de mesures ;

- le transducteur ultrasonore est circulaire avec un diamètre compris entre 2 mm et 15 mm ;

- le transducteur ultrasonore possède une fréquence de fonctionnement comprise entre 1 MHz et 15 MHz ;

- la sonde est configurée pour calculer un paramètre d'atténuation ultrasonore moyen, ledit paramètre d'atténuation ultrasonore moyen étant calculé à partir d'une multiplicité de paramètres d'atténuation ultrasonore instantanés et de coefficients de qualités associés aux paramètres d'atténuation ultrasonore instantanés ;

- le transducteur ultrasonore est une sonde abdominale convexe.

Un autre objet de la présente invention est un dispositif pour élastographie hybride mettant en oeuvre le procédé d'élastographie hybride selon l'invention.
Un tel dispositif hybride selon l'invention comprend :

- Une sonde selon l'invention ;
- Une unité centrale connectée à la sonde et comprenant au moins des moyens de calcul pour le traitement des signaux ultrasonores réfléchis, des moyens d'affichage et des moyens de contrôle et/ou de saisie.

Dans une mise en oeuvre particulière, l'unité centrale est placée à l'intérieur de la sonde.

## LISTE DES FIGURES

D'autres caractéristiques et avantages de l'invention ressortiront clairement de la description qui en est donnée ci-dessous, à titre indicatif et nullement limitatif, en référence aux figures parmi lesquelles :

- La figure 1 illustre les étapes du procédé d'élastographie hybride selon l'invention ;
- La figure 2 montre schématiquement les vibrations appliquées par le vibreur et les acquisitions ultrasonores lors de la mise en oeuvre du procédé selon l'invention illustré à la figure 1 ;
- La figure 3 montre schématiquement un mode particulier de réalisation du procédé d'élastographie illustré à la figure 1, dit mode stroboscopique ;
- La figure 4 montre les résultats obtenus en mettant en oeuvre la partie du procédé selon l'invention relative au positionnement du vibreur ;
- La figure 5 représente une sonde pour élastographie hybride selon l'invention ;
- La figure 6a représente un mode particulier de réalisation de la sonde pour élastographie hybride selon l'invention ;
- La figure 6b représente un dispositif pour élastographie hybride selon l'invention.

## DESCRIPTION DETAILLEE

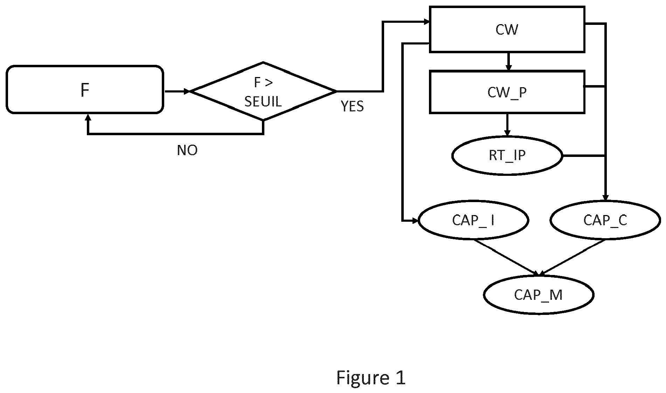

La figure 1 illustre les étapes du procédé d'élastographie hybride P selon l'invention.
Le procédé P selon l'invention comprend une étape CW d'application d'une vibration continue basse fréquence à l'aide d'un vibreur compris dans une sonde au contact du milieu viscoélastique.
La fréquence centrale de la vibration continue est comprise entre 5 et 500 Hz.
L'étape CW du procédé P comprend en outre la génération, par le transducteur ultrasonore, d'une série d'acquisitions ultrasonores. La série d'acquisitions ultrasonores comprend des groupes d'acquisitions ultrasonores. Les groupes d'acquisitions ultrasonores sont émis avec un taux de répétition compris entre 5 Hz et 500 Hz, chaque groupe comprenant au moins une acquisition ultrasonore.
Le taux de répétition des groupes ultrasonores est également appelé taux de répétition inter-groupe.
Une acquisition ultrasonore comprend l'émission d'un tir ultrasonore suivie par la détection et l'enregistrement des signaux ultrasonores réfléchis ou échos.
L'application d'une vibration continue au milieu viscoélastique génère une onde élastique à l'intérieur dudit milieu. L'onde élastique comprend une superposition d'ondes de cisaillement et d'ondes de compression. L'étude des propriétés de cette onde élastique permet d'obtenir des informations concernant le bon positionnement de la sonde au regard du milieu viscoélastique.
Le milieu viscoélastique à caractériser diffuse au moins partiellement les tirs ultrasonores. Il est donc possible de détecter les signaux ultrasonores réfléchis lors de l'émission de la première série d'acquisitions ultrasonores.
La détection des signaux ultrasonores réfléchis peut être réalisée à l'aide du même transducteur ultrasonore utilisé pour l'émission.
Un paramètre d'atténuation ultrasonore peut être déterminé à partir des signaux ultrasonores réfléchis. Par exemple la valeur CAP_I de l'atténuation ultrasonore correspondante à une acquisition ultrasonore donnée peut être déterminée. La valeur CAP_I est également appelée valeur individuelle ou instantanée de l'atténuation ultrasonore ou paramètre instantané d'atténuation ultrasonore.

Les signaux ultrasonores réfléchis détectés lors de l'étape CW sont successivement traités lors d'une étape de détermination d'au moins une propriété de la propagation de l'onde élastique à l'intérieur du milieu viscoélastique CW_P.

Typiquement, lors de cette étape, les signaux ultrasonores réfléchis sont corrélés entre eux de sorte à mesurer les déplacements du milieu viscoélastique engendrés par l'onde élastique générée par l'application de la vibration continue, selon une technique connue dans le domaine de l'élastographie et plus généralement des ultrasons.

A partir des déplacements mesurés à l'intérieur du milieu viscoélastique, il est possible de calculer des propriétés de l'onde élastique telles que l'amplitude et la phase en fonction de la position à l'intérieur du milieu viscoélastique. La position d'un point à l'intérieur du milieu viscoélastique est mesurée comme la distance entre le transducteur ultrasonore et ledit point calculée le long de la direction de propagation des ultrasons émis par le transducteur. Pour cette raison la position d'un point à l'intérieur du milieu viscoélastique est généralement appelée profondeur.

Il est également possible de déterminer d'autres paramètres de l'onde élastique à l'intérieur du milieu viscoélastique, tels que la vitesse de phase ou l'atténuation de l'onde élastique.

Les variations de l'amplitude et de la phase de l'onde élastique en fonction de la profondeur à l'intérieur du tissu peuvent être calculées. En réalisant un ajustement entre le modèle théorique et les propriétés mesurées, il est possible d'extraire un paramètre de qualité de l'ajustement. A partir de ce paramètre de qualité de l'ajustement et/ou des autres propriétés de l'onde élastique, il est possible de calculer un indicateur de positionnement RT_IP de la sonde par rapport au tissu à caractériser.

Par exemple, un des modèles théoriques utilisés prévoit une variation linéaire du retard de phase à la fréquence centrale de l'onde élastique avec la profondeur dans le milieu à caractériser. Dans ce cas l'ajustement est un ajustement linéaire et le paramètre de qualité de l'ajustement traduit la linéarité de la phase en fonction de la profondeur dans le milieu. Un indicateur possible est le coefficient de détermination $R^2$ donnant la qualité de la prédiction de la régression linéaire de la courbe du retard de phase en fonction de la profondeur dans la gamme de profondeur étudiée.

Selon un mode de réalisation l'étape CW_P de détermination d'au moins une propriété de l'onde élastique à l'intérieur du tissu est effectuée en même temps que l'étape d'application de la vibration continue CW et de détection des premiers signaux ultrasonores réfléchis.

Grâce au procédé P selon l'invention il est donc possible de mesurer en temps réel les propriétés de l'onde élastique à l'intérieur du tissu et d'obtenir en temps réel l'indicateur de positionnement de la sonde RT_IP.

Avantageusement, un taux de répétition faible permet de réduire la taille des données enregistrées lors de l'étape de génération de la série d'acquisitions ultrasonores CW et de traiter ces données en temps réel pour obtenir l'indicateur de positionnement RT_IP. La valeur de cet indicateur est typiquement comprise entre 0 et 1. La valeur 0 correspond à un indicateur mauvais et la valeur 1 à un indicateur bon.

Avantageusement, l'indicateur de positionnement est fourni à l'opérateur pour l'aider à trouver un point de mesure satisfaisant. Il peut être fourni par exemple (liste non exhaustive) sous la forme d'un affichage d'un indicateur coloré, sous la forme d'une barre plus ou moins longue, etc.

Un coefficient de qualité de la mesure de l'atténuation ultrasonore, CAP_C, est également calculé à partir du signal ultrasonore. La valeur de ce coefficient est typiquement comprise entre 0 et 1. La valeur 0 correspond à une qualité faible et la valeur 1 à une qualité élevée.

Le coefficient CAP_C est associé à la valeur individuelle de l'atténuation ultrasonore CAP_I obtenue à partir des données ultrasonores en cours d'acquisition.

Le coefficient de qualité CAP_C peut par exemple être calculé à partir des seules propriétés du signal ultrasonore. Il peut également être une combinaison de la qualité du signal ultrasonore et de propriétés de l'onde élastique.

Selon un mode de réalisation la valeur individuelle de l'atténuation ultrasonore n'est conservée que si l'indicateur de positionnement RT_IP est bon. Dans le cas où l'indicateur de positionnement RT_IP est mauvais, le coefficient CAP_C correspondant est par exemple mis à zéro.

Selon un mode de réalisation, la vibration continue est déclenchée uniquement si la force de contact F entre le vibreur et le tissu viscoélastique est supérieure à un seuil inférieur prédéterminé. La valeur du seuil est typiquement de 1 N.

Avantageusement, ce seuil inférieur assure un couplage suffisant entre la sonde et le milieu viscoélastique.

Selon un mode de réalisation, la vibration continue est déclenchée uniquement si la force de contact F entre le vibreur et le tissu viscoélastique est inférieure à un seuil supérieur prédéterminé. La valeur du seuil est typiquement de 10 N.

Avantageusement, ce seuil supérieur assure que la vibration n'est pas déformée et que le milieu étudié n'est pas abîmé.

A cause du mouvement vibratoire continu du vibreur, la détermination de la force F de contact entre le vibreur et le milieu est plus complexe que dans le cas d'un procédé d'élastographie transitoire standard. En présence de la vibration continue basse fréquence, la force de contact entre le vibreur et le milieu viscoélastique est donnée par la formule suivante :

$$F = k\left(x + A \times cos(2\pi f_{low} t)\right)$$

Dans cette formule x est le déplacement du vibreur, k la constante élastique du ressort placé dans la sonde, A l'amplitude de la vibration continue et $f_{low}$ la fréquence de la vibration continue.

La force F peut être mesurée à l'aide d'un capteur de force placé sur la sonde pour élastographie hybride. Successivement en appliquant un filtre passe bas au signal ainsi mesuré, il est possible d'éliminer la partie basse fréquence et de déduire la force moyenne de contact :

$$F_{moyenne} = k(x)$$

Avantageusement la valeur de la force moyenne appliquée est fournie à l'opérateur pour qu'il l'adapte afin que la vibration basse fréquence et l'acquisition de données se poursuivent.

Avantageusement les valeurs individuelles CAP_I sont accumulées dans une mémoire et utilisées pour calculer une valeur moyenne CAP_M. CAP_M peut être calculée de plusieurs manières. Par exemple

$$CAP\_M = \frac{\sum_{i=1}^{N}(CAP\_I(i) \times CAP\_C(i))}{\sum_{i=1}^{N}(CAP\_C(i))}$$

Les valeurs de CAP_C sont alors utilisées pour pondérer les valeurs individuelles CAP_I mesurées. La valeur CAP_M est conservée en fin d'examen comme étant la valeur d'atténuation ultrasonore mesurée. L'unité de la valeur CAP_M est par exemple le dB/m.

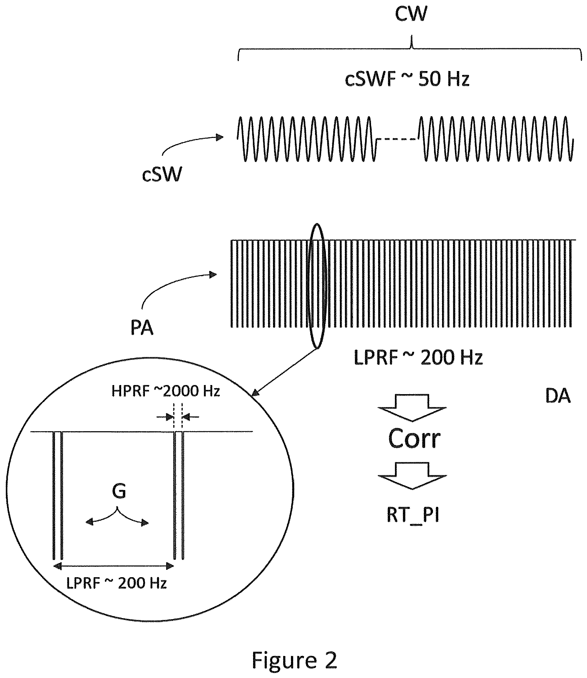

La figure 2 montre schématiquement :

- La vibration continue basse fréquence cSW appliquée par le vibreur lors de l'étape CW illustrée à la figure 1 ;
- La série d'acquisitions ultrasonores PA formée par des groupes G d'acquisitions et générée par le transducteur ultrasonore lors de l'étape CW illustrée à la figure 1.

Lors de l'étape d'application d'une vibration continue CW, le vibreur oscille à une fréquence comprise entre 5 et 500 Hz, avec une amplitude comprise entre 10 µm et 5 mm.

Avantageusement, grâce à la faible amplitude et à la faible fréquence de la vibration continue un opérateur peut facilement maintenir la sonde au contact du milieu viscoélastique.

En même temps que l'application de la vibration continue basse fréquence, le transducteur ultrasonore émet des acquisitions ultrasonores PA formée par des groupes G d'acquisitions ultrasonores. Dans l'exemple illustré à la figure 2 chaque groupe G comprend deux acquisitions ultrasonores.

Les groupes G d'acquisitions ultrasonores sont émis avec un taux de répétition LPRF compris entre 10 Hz et 500 Hz ou taux de répétition inter-groupe ou siplement taux de répétition. Les acquisitions ultrasonores appartenant à un même groupe G sont émises avec un taux de répétition intra-groupe HPRF compris entre compris entre 500 Hz et 10 kHz.

Le transducteur ultrasonore détecte également les signaux ultrasonores réfléchis lors de la génération des acquisitions ultrasonores PA, comme expliqué en référence à l'étape CW illustré à la figure 1. A partir de la première série d'acquisitions ultrasonores PA, il est possible de calculer, par une étape de corrélation CORR entre signaux ultrasonores appartenant au même groupe G, les déplacements du milieu viscoélastique. Lesdits déplacements du milieu viscoélastique sont engendrés par la propagation de l'onde élastique générée par la vibration continue appliquée par le vibreur.

Il est important de noter qu'il existe un grand nombre de séquences ultrasonores possibles pour la mise en oeuvre de ce procédé et que les éléments indiqués ne constituent aucunement une liste exhaustive du champ des possibles.

Avantageusement, en appliquant une technique de corrélation aux acquisitions ultrasonores appartenant à un même groupe G - et donc rapprochées temporellement - il est possible de détecter des déplacements petits et de l'ordre de 1 µm à 10 µm.

Comme expliqué en référence à l'étape CW_P illustrée à la figure 1, les déplacements du milieu viscoélastique sont ensuite utilisés pour calculer des propriétés de l'onde élastique telles que son amplitude et sa phase en fonction de la profondeur dans le milieu. En comparant les propriétés mesurées avec un modèle théorique il est possible de déduire en temps réel un indicateur de positionnement RT_IP.

Par exemple, l'indicateur de positionnement peut être lié à la linéarité de la phase de l'onde élastique en fonction de la profondeur dans le milieu à caractériser. L'indicateur dépend alors de la qualité de l'ajustement de l'évolution de la phase en fonction de la profondeur par une droite.

Par exemple, l'indicateur de positionnement peut être lié à la décroissance de l'amplitude de l'onde élastique en fonction de la profondeur dans le milieu à caractériser. L'indicateur dépend alors de la qualité du fit en $1/Z^n$ où $Z$ est la profondeur et $n$ un coefficient entier compris entre 1 et 3.

Par exemple, la valeur de l'indicateur de positionnement temps réel RT_IP est comprise entre 0 et 1, avec des valeurs proches de 1 si la sonde est bien positionnée par rapport au milieu viscoélastique d'intérêt.

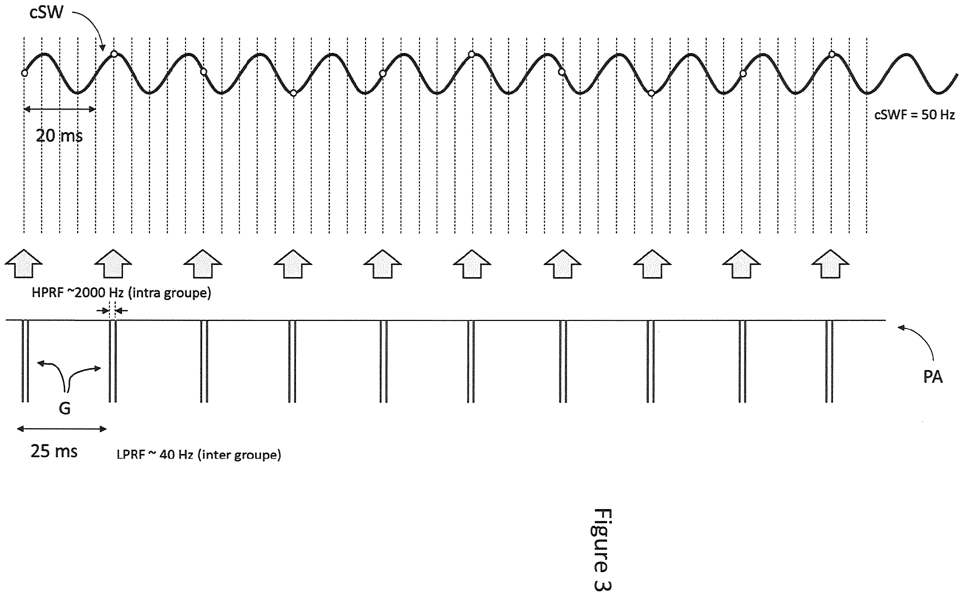

La figure 3 montre un mode de réalisation particulier des étapes CW et CW_P du procédé P selon l'invention, dit mode stroboscopique.

La ligne sinusoïdale en trait continu représente schématiquement la vibration continue cSW appliquée par le premier vibreur. La vibration continue cSW a par exemple une fréquence centrale cSWF de 50 Hz correspondant à une période de 20 ms.

Les traits verticaux en continu représentent les groupes G d'acquisitions ultrasonores formant la première série d'acquisitions ultrasonores PA. Les groupes G sont émis avec un premier taux de répétition LPRF. Selon le mode d'acquisition stroboscopique, le premier taux de répétition LPRF est plus petit que la fréquence centrale de la vibration continue cSWF.

Le taux de répétition intra-groupe est compris entre 500 Hz et 100 kHz, ce qui permet de mesurer des déplacements petits de l'ordre de 1 $\mu$m.

Les cercles blancs et les flèches le long de la vibration continue cSW correspondent aux échantillonnages effectués par chaque groupe G d'acquisitions ultrasonores.

Grâce au fait que le taux de répétition LPRF des groupes G est inférieur à la fréquence centrale de la vibration continue cSW, il est possible d'échantillonner de façon complète la vibration continue cSW au bout de quelques périodes d'oscillations, comme il est illustré par les cercles blancs.

Avantageusement, le mode stroboscopique permet d'échantillonner de façon complète la vibration continue cSW tout en utilisant un premier taux de répétition LPRF faible. L'utilisation d'un taux de répétition faible permet de traiter les signaux réfléchis en temps réel et donc d'obtenir l'indicateur de positionnement RT_IP en temps réel.

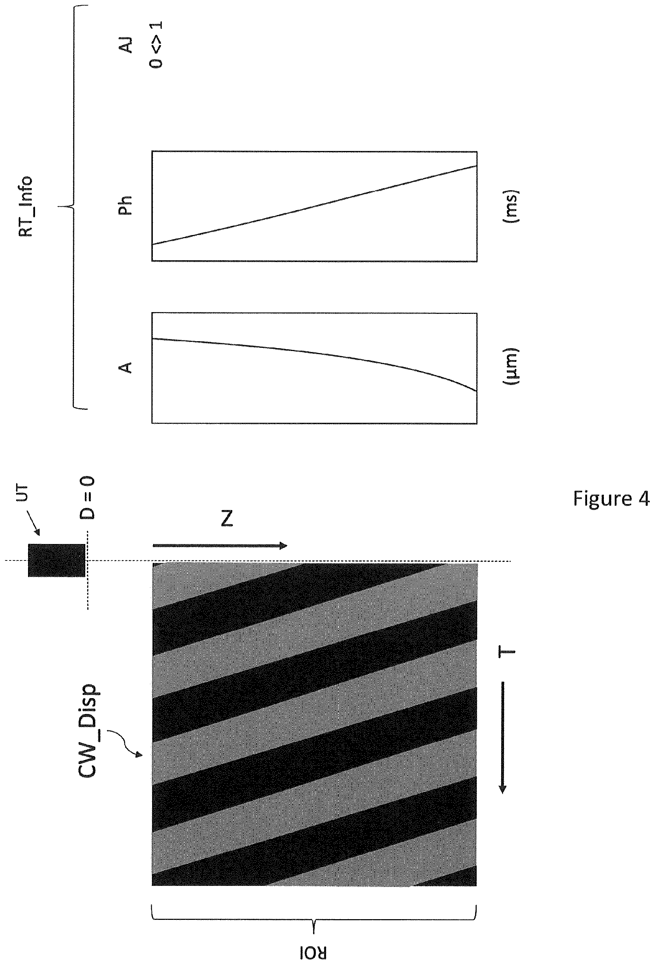

La figure 4 montre schématiquement les résultats obtenus en mettant en oeuvre la partie du procédé P selon l'invention relative au positionnement du vibreur.

Le graphe CW_Disp montre le déplacement (ou tout autre paramètre de mouvement comme la vitesse, la déformation, le taux de déformation) du milieu viscoélastique dans une région d'intérêt ROI en fonction de la profondeur Z dans le milieu et du temps T. Les déplacements sont représentés en utilisant une échelle en fausses couleurs, les couleurs moins foncées représentant un déplacement selon la direction positive de l'axe D. Les déplacements sont causés par la vibration basse fréquence continue appliquée par le vibreur et sont mesurés par le transducteur ultrasonore UT placé au contact de la surface du milieu, en Z=0.

A partir des déplacements mesurés CW_Disp dans la région d'intérêt ROI à l'intérieur du milieu viscoélastique, il est possible d'extraire en temps réel des informations RT _Info concernant l'onde élastique se propageant à l'intérieur du milieu et générée par la vibration continue. Des exemples de telles propriétés sont l'amplitude A et la phase Ph de l'onde élastique en fonction de la profondeur à l'intérieur du milieu.

En comparant les valeurs de A et Ph mesurées avec des seuils prédéterminés il est possible de déterminer un indicateur de positionnement du vibreur par rapport au milieu viscoélastique.

Alternativement, il est possible d'obtenir un paramètre de qualité de l'ajustement AJ entre les grandeurs A et Ph mesurées et un modèle théorique décrivant l'amplitude et phase d'une onde élastique se propageant à l'intérieur du milieu. Dans ce cas, l'indicateur de positionnement est obtenu à partir du paramètre de qualité de l'ajustement AJ. Par exemple, un paramètre de qualité de l'ajustement est le coefficient de détermination $R^2$ donnant la qualité de la prédiction de la régression linéaire de la courbe du retard de phase en fonction de la profondeur dans la gamme de profondeur étudiée.

Selon un mode de réalisation, le paramètre de qualité de l'ajustement AJ est compris entre 0 et 1.

Une fois calculé, l'indicateur de positionnement peut être affiché sous forme d'un chiffre ou d'une lettre ou en utilisant une échelle de couleurs. Alternativement, l'indicateur de positionnement peut être une simple indication visuelle de type disque colorée. Alternativement, l'indicateur de positionnement peut être une simple indication visuelle de type « Positionnement OK » indiquant que l'opérateur peut déclencher l'étape d'élastographie transitoire.

Selon un mode de réalisation, la vitesse de la propagation de l'onde élastique est conservée comme une mesure de l'élasticité du milieu.

Lors de la mise en oeuvre du procédé P selon l'invention, les graphes CW_Disp, RT_Info et l'indicateur de positionnement du vibreur sont calculés et affichés de façon concomitante.

Avantageusement, grâce à la structure de la série d'acquisitions ultrasonores, l'indicateur de positionnement RT_IP ainsi que le graphe RT_Info peuvent être calculés et affichés en temps réel.

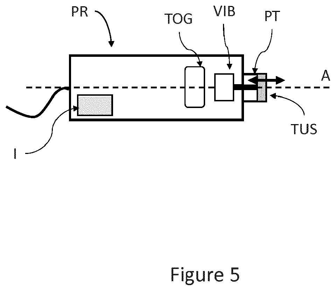

La figure 5 représente schématiquement une sonde PR pour la mesure de l'atténuation ultrasonore guidée par élastographie harmonique.

La sonde PR comprend :

- Un vibreur VIB configuré pour appliquer au milieu viscoélastique une vibration continue basse fréquence, la vibration continue basse fréquence générant une onde élastique à l'intérieur du milieu viscoélastique ;
- Un transducteur ultrasonore TUS configuré pour émettre une série d'acquisitions ultrasonores, ladite série d'acquisitions ultrasonores comprenant des groupes d'acquisitions ultrasonores, les groupes d'acquisitions ultrasonores étant générés avec un taux de répétition, chaque groupe d'acquisitions ultrasonores comprenant au moins une acquisition ;

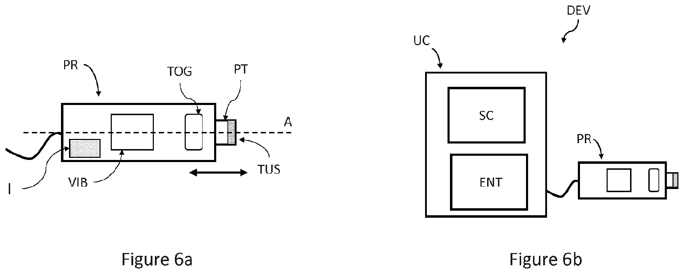

Selon le mode de réalisation illustré à la figure 6, le transducteur ultrasonore TUS est monté sur l'axe du vibreur VIB.

Selon un mode de réalisation, la sonde PR comprend des moyens de calcul pour calculer en temps réel l'indicateur de positionnement RT_IP à partir des acquisitions ultrasonores.

Selon un mode de réalisation, la sonde PR comprend des moyens de calcul et d'affichage I de l'indicateur de positionnement temps réel RT_IP.

Selon un mode de réalisation, le taux de rafraîchissement de l'affichage de l'indicateur de positionnement est supérieur à 5 Hz.

Avantageusement, l'affichage de l'indicateur de positionnement temps réel au niveau de la sonde permet à l'opérateur d'optimiser le positionnement de la sonde sans détourner son regard de la sonde et du corps du patient. Cela simplifie l'opération de positionnement de la sonde.

Selon un mode de réalisation, le transducteur ultrasonore TUS peut être fixé au corps de la sonde à l'aide d'une pointe PT.

Le vibreur VIB fait osciller la sonde PR. Au cours de cette oscillation, le transducteur ultrasonore TUS est poussé contre le milieu viscoélastique appliquant la vibration continue basse fréquence et engendrant l'onde élastique à l'intérieur du milieu.

Selon un mode de réalisation, le vibreur VIB pour l'application de la vibration basse fréquence comprend un anneau vibratoire placé autour du transducteur ultrasonore TUS ou autour de la pointe de sonde PT.

Selon un mode de réalisation, la pointe de sonde PT est mobile et peut être actionnée par le vibreur VIB. Le transducteur ultrasonore TUS est alors poussé contre le milieu viscoélastique pour appliquer la vibration, selon la direction de la flèche de figure 6.

Selon un deuxième mode de réalisation, illustré à la figure 7a, la sonde PR est une sonde inertielle sans parties mobiles. Dans ce cas, le mouvement du vibreur VIB à l'intérieur de la sonde PR entraîne le mouvement de la sonde et la vibration continue ou impulsionnelle est à nouveau appliquée en poussant le transducteur TUS contre le milieu viscoélastique.

L'axe de mouvement du vibreur A est de préférence un axe de symétrie du transducteur ultrasonore TUS. Par exemple, le transducteur ultrasonore TUS peut avoir une section circulaire, l'axe A passant par le centre du transducteur ultrasonore TUS.

Selon un mode de réalisation, la sonde PR comprend des moyens de contrôle TOG pour déclencher la mesure.

La sonde PR selon la figure 6a comprend donc un vibreur destiné à appliquer la vibration continue basse fréquence.

Selon un mode de réalisation, le diamètre du transducteur ultrasonore est compris entre 2 et 15 mm.

Selon un mode de réalisation la fréquence centrale du transducteur ultrasonore est comprise entre 1 MHz et 15 MHz.

Selon un mode de réalisation le transducteur ultrasonore TUS est une sonde abdominale convexe.

Selon un mode de réalisation, la sonde comprend un indicateur de positionnement qui se déclenche lorsque la sonde est correctement positionnée. Cet indicateur peut être un indicateur visuel, par exemple un changement de couleur des diodes. Alternativement, l'indicateur peut être un indicateur sonore ou haptique tel qu'un changement de type ou d'amplitude d'une vibration.

La figure 6b illustre un dispositif d'élastographie hybride selon l'invention DEV.

Le dispositif DEV selon l'invention comprend :

- Une sonde PR selon l'invention ;
- Une unité centrale UC connectée à la sonde PR.

L'unité centrale peut comporter :

- Des moyens de calcul pour le traitement des signaux ultrasonores réfléchis ;
- Un écran SC pour l'affichage des résultats obtenus aux différentes étapes du procédé P selon l'invention ;
- Des moyens de contrôle ou de saisie ENT pour le contrôle du dispositif de la part de l'opérateur.

L'unité centrale UC peut être reliée à la sonde PR par une liaison filaire ou par des moyens de communication sans fils.

Selon un mode de réalisation l'écran SC est adapté pour l'affichage des résultats illustrés à la figure 5. L'écran SC peut également afficher en temps réel l'indicateur RT_IP de position calculé lors de l'étape CW_P du procédé P selon l'invention.

Selon un mode de réalisation, l'unité centrale comprend des moyens configurés pour déclencher automatiquement l'application d'une impulsion basse fréquence sur la base de la valeur de l'indicateur de positionnement RT_IP calculé et affiché en temps réel.

Selon un mode de réalisation, l'unité centrale est comprise dans la sonde PR.

## Revendications

1. Procédé de mesure d'un paramètre d'atténuation ultrasonore guidé par élastographie harmonique comprenant une étape d'application, à l'aide d'un vibreur compris dans une sonde au contact d'un milieu viscoélastique, d'une vibration continue basse fréquence, la vibration continue basse fréquence générant une onde élastique à l'intérieur du milieu viscoélastique et de génération (CW), pendant la propagation de l'onde élastique, à l'aide d'un transducteur ultrasonore au contact du milieu viscoélastique, d'une série d'acquisitions ultrasonores, ladite série d'acquisitions ultrasonores comprenant des groupes d'acquisitions ultrasonores, les groupes d'acquisitions ultrasonores étant générés avec un taux de répétition (LPRF), chaque groupe d'acquisitions ultrasonores comprenant au moins une acquisition ;

   le paramètre d'atténuation ultrasonore étant mesuré à partir des acquisitions ultrasonores;
   ledit procédé comprenant en outre une étape de détermination (CW_P), à partir de la série d'acquisitions ultrasonores, d'au moins une propriété de la propagation de l'onde élastique à l'intérieur du milieu viscoélastique, et d'utilisation de la propriété de la propagation de l'onde élastique à l'intérieur du milieu viscoélastique pour calculer un indicateur de positionnement temps réel (RT_IP) de la sonde par rapport au milieu viscoélastique à étudier.

2. Procédé de mesure d'un paramètre d'atténuation ultrasonore guidé par élastographie harmonique selon la revendication précédente **caractérisé en ce qu'**il comprend en outre une étape d'affichage en temps réel de l'indicateur de positionnement temps réel (RT_IP).

3. Procédé de mesure d'un paramètre d'atténuation ultrasonore guidé par élastographie harmonique selon l'une des revendications 1 à 2 **caractérisé en ce que** la propriété de la propagation de l'onde élastique mesurée est choisie parmi l'amplitude de l'onde élastique, la phase de l'onde élastique, la vitesse de phase de l'onde élastique, l'élasticité du milieu viscoélastique, le module d'Young du milieu viscoélastique et le module de cisaillement du milieu viscoélastique.

4. Procédé de mesure d'un paramètre d'atténuation ultrasonore guidé par élastographie harmonique selon l'une des revendications précédentes **caractérisé en ce que** l'étape d'application d'une vibration continue basse fréquence est déclenchée uniquement si la force de contact entre le vibreur et le milieu viscoélastique est supérieure à un seuil inférieur prédéterminé.

5. Procédé de mesure d'un paramètre d'atténuation ultrasonore guidé par élastographie harmonique selon l'une des revendications précédentes **caractérisé en ce que** l'étape d'application d'une vibration continue basse fréquence est déclenchée uniquement si la force de contact entre le vibreur et le milieu viscoélastique est supérieur à un seuil inférieur et inférieur à un seuil supérieur prédéterminés.

6. Procédé de mesure d'un paramètre d'atténuation ultrasonore guidé par élastographie harmonique selon l'une des revendications précédentes **caractérisé en ce que** la série d'acquisitions ultrasonores est formée par une répétition de groupes comprenant au moins deux acquisitions ultrasonores ayant un taux de répétition intra-groupe (HPRF) compris entre 500 Hz et 10 kHz et un taux de répétition (LPRF) compris entre 10 Hz et 10 kHz.

7. Procédé de mesure d'un paramètre d'atténuation ultrasonore guidé par élastographie harmonique selon l'une des revendications précédentes **caractérisé en ce que** le taux de répétition (LPRF) est plus faible que la fréquence de la vibration continue (cSWF).

8. Procédé de mesure d'un paramètre d'atténuation ultrasonore guidé par élastographie harmonique selon l'une des revendications précédentes **caractérisé en ce que** le paramètre d'atténuation ultrasonore est un paramètre instantané et un coefficient de qualité associé à la mesure du paramètre d'atténuation ultrasonore instantané est calculé à partir d'une propriété de la propagation de l'onde élastique à l'intérieur du milieu viscoélastique et/ou des propriétés de l'onde ultrasonore.

9. Procédé de mesure d'un paramètre d'atténuation ultrasonore guidé par élastographie harmonique selon la revendication précédente **caractérisé en ce qu'**un paramètre d'atténuation ultrasonore moyen est calculé à partir d'une pluralité de paramètres d'atténuation ultrasonore instantanés et des coefficients de qualités associés à chaque paramètre d'atténuation ultrasonore instantané.

10. Sonde de mesure d'un paramètre d'atténuation ultrasonore guidée par élastographie harmonique comprenant :

   - Un vibreur configuré pour appliquer au milieu viscoélastique une vibration continue basse fréquence, la vibration continue basse fréquence générant une onde élastique à l'intérieur du milieu viscoélastique ;
   - Un transducteur ultrasonore configuré pour émettre une série d'acquisitions ultrasonores, ladite série d'acquisitions ultrasonores comprenant des groupes d'acquisitions ultrasonores, les groupes d'acquisitions ultrasonores étant générés avec un taux de répétition, chaque groupe d'acquisitions ultrasonores comprenant au moins une acquisition ;
   - Des moyens de calcul et d'affichage en temps réel d'un indicateur de positionnement de la sonde, ledit indicateur de positionnement étant calculé à partir d'une propriété de la propagation de l'onde élastique, ladite propriété de propagation de l'onde élastique étant déterminée à partir de la série d'acquisitions ultrasonores ;

   le paramètre d'atténuation ultrasonore étant mesuré à partir des acquisitions ultrasonores réalisées pendant l'application de la vibration continue basse fréquence.

11. Sonde de mesure d'un paramètre d'atténuation ultrasonore guidée par élastographie harmonique selon la revendication précédente ladite sonde étant en outre configurée pour appliquer la vibration continue lorsque la force de contact entre la sonde et le milieu viscoélastique est supérieure à une valeur prédéterminée.

12. Sonde de mesure d'atténuation ultrasonore guidée par élastographie harmonique selon la revendication 10 ou 11 **caractérisée en ce que** le transducteur est porté par le vibreur.

13. Sonde de mesure d'atténuation ultrasonore guidée par élastographie harmonique selon une des revendications 11 ou 12 configurée pour calculer un paramètre d'atténuation ultrasonore moyen, ledit paramètre d'atténuation ultrasonore moyen étant calculé à partir d'une multiplicité de paramètres d'atténuation ultrasonore instantanés et de coefficients de qualités associés aux paramètres d'atténuation ultrasonore instantanés.

14. Dispositif de mesure d'atténuation ultrasonore guidé par élastographie harmonique comprenant :

   - Une sonde (PR) pour la mesure d'atténuation ultrasonore guidée par élastographie harmonique selon la revendication 10 ou 11;
   - Une unité centrale (UC) connectée à la sonde (PR) et comprenant au moins des moyens de calcul pour le traitement des signaux ultrasonores réfléchis, des moyens d'affichage (SC) et des moyens de contrôle et/ou de saisie (ENT).

**Patentansprüche**

1. Verfahren zum Messen eines durch harmonische Elastographie geführten Ultraschalldämpfungsparameters, das einen Schritt umfasst, bei dem mit Hilfe eines Vibrators, der in einer Sonde enthalten ist, die mit einem viskoelastischen Medium in Kontakt ist, eine niederfrequente kontinuierliche Schwingung angelegt wird, wobei die niederfrequente kontinuierliche Schwingung eine elastische Welle innerhalb des viskoelastischen Mediums erzeugt, sowie einen Schritt des Generierens (CW) einer Reihe von Ultraschallerfassungen während der Ausbreitung der elastischen Welle mit Hilfe eines Ultraschallwandlers, der mit dem viskoelastischen Medium in Kontakt steht, wobei die Reihe von Ultraschallerfassungen Gruppen von Ultraschallerfassungen umfasst, die mit einer Wiederholungsrate (LPRF) erzeugt werden und jede Gruppe von Ultraschallerfassungen mindestens eine Erfassung umfasst; wobei der Ultraschalldämpfungsparameter anhand der Ultraschallerfassungen gemessen wird; wobei das Verfahren ferner einen Schritt umfasst, bei dem aus der Reihe von Ultraschallerfassungen mindestens eine Eigenschaft der Ausbreitung der elastischen Welle innerhalb des viskoelastischen Mediums bestimmt wird (CW_P) und die Eigenschaft der Ausbreitung der elastischen Welle innerhalb des viskoelastischen Mediums verwendet wird, um einen Echtzeit-Positionsindikator (RT _IP) der Sonde in Bezug auf das zu untersuchende viskoelastische Medium zu berechnen.

2. Verfahren zum Messen eines durch harmonische Elastographie geführten Ultraschalldämpfungsparameters nach dem vorhergehenden Anspruch, **dadurch gekennzeichnet, dass** es außerdem einen Schritt der Echtzeitanzeige des Echtzeit-Positionsanzeigers (RT_IP) umfasst.

3. Verfahren zum Messen eines durch harmonische Elastographie geführten Ultraschalldämpfungsparameters nach einem der Ansprüche 1 bis 2, **dadurch gekennzeichnet, dass** die gemessene Eigenschaft der Ausbreitung der elastischen Welle ausgewählt ist aus der Amplitude der elastischen Welle, der Phase der elastischen Welle, der Phasengeschwindigkeit der elastischen Welle, der Elastizität des viskoelastischen Mediums, dem Young-Modul des viskoelastischen Mediums und dem Schermodul des viskoelastischen Mediums.

4. Verfahren zum Messen eines durch harmonische Elastographie geführten Ultraschalldämpfungsparameters nach einem der vorhergehenden Ansprüche, **dadurch gekennzeichnet, dass** der Schritt des Anlegens einer niederfrequenten kontinuierlichen Vibration nur dann ausgelöst wird, wenn die Kontaktkraft zwischen dem Vibrator und dem viskoelastischen Medium größer als ein vorbestimmter unterer Schwellenwert ist.

5. Verfahren zum Messen eines durch harmonische Elastographie geführten Ultraschalldämpfungsparameters nach einem der vorhergehenden Ansprüche, **dadurch gekennzeichnet, dass** der Schritt des Anlegens einer niederfrequenten kontinuierlichen Vibration nur dann ausgelöst wird, wenn die Kontaktkraft zwischen dem Vibrator und dem viskoelastischen Medium größer als ein vorbestimmter unterer Schwellenwert und kleiner als ein vorbestimmter oberer Schwellenwert ist.

6. Verfahren zum Messen eines durch harmonische Elastographie geführten Ultraschalldämpfungsparameters nach einem der vorhergehenden Ansprüche, **dadurch gekennzeichnet, dass** die Reihe von Ultraschallerfassungen durch eine Gruppenwiederholung gebildet wird, die mindestens zwei Ultraschallerfassungen mit einer gruppeninternen Wiederholungsrate (HPRF) zwischen 500 Hz und 10 kHz und einer Wiederholungsrate (LPRF) zwischen 10 Hz und 10 kHz umfasst.

7. Verfahren zum Messen eines durch harmonische Elastographie geführten Ultraschalldämpfungsparameters nach einem der vorhergehenden Ansprüche, **dadurch gekennzeichnet, dass** die Wiederholungsrate (LPRF) kleiner ist als die Frequenz der kontinuierlichen Vibration (cSWF).

8. Verfahren zum Messen eines durch harmonische Elastographie geführten Ultraschalldämpfungsparameters nach einem der vorhergehenden Ansprüche, **dadurch gekennzeichnet, dass** der Ultraschalldämpfungsparameter ein momentaner Parameter ist und dass ein Qualitätskoeffizient, der mit der Messung des momentanen Ultraschalldämpfungsparameters verbunden ist, aus einer Eigenschaft der Ausbreitung der elastischen Welle innerhalb des viskoelastischen Mediums und/oder den Eigenschaften der Ultraschallwelle berechnet wird.

9. Verfahren zum Messen eines durch harmonische Elastographie geführten Ultraschalldämpfungsparameters nach dem vorhergehenden Anspruch, **dadurch gekennzeichnet, dass** ein mittlerer Ultraschalldämpfungsparameter aus einer Vielzahl von momentanen Ultraschalldämpfungsparametern und den mit jedem momentanen Ultraschalldämpfungsparameter verbundenen Qualitätskoeffizienten berechnet wird.

10. Sonde zur Messung eines durch harmonische Elastographie geführten Ultraschalldämpfungsparameters, umfassend:

- Einen Vibrator, der so konfiguriert ist, dass er am viskoelastischen Medium eine niederfrequente kontinuierliche Vibration anlegt, wobei die niederfrequente kontinuierliche Vibration eine elastische Welle innerhalb des viskoelastischen Mediums erzeugt;

- Einen Ultraschallwandler, der so konfiguriert ist, dass er eine Reihe von Ultraschallerfassungen aussendet, wobei die Reihe von Ultraschallerfassungen Gruppen von Ultraschallerfassungen umfasst, die mit einer Wiederholungsrate erzeugt werden, wobei jede Gruppe von Ultraschallerfassungen mindestens eine Erfassung umfasst;

- Mittel zur Berechnung und Anzeige eines Positionierungsindikators der Sonde in Echtzeit, wobei der Positionierungsindikator anhand einer Eigenschaft der Ausbreitung der elastischen Welle berechnet wird, wobei die Eigenschaft der Ausbreitung der elastischen Welle anhand der Reihe von Ultraschallerfassungen bestimmt wird; wobei der Ultraschalldämpfungsparameter anhand der Ultraschallerfassungen gemessen wird, die während der Anwendung der niederfrequenten kontinuierlichen Vibration durchgeführt werden.

11. Sonde zur Messung eines durch harmonische Elastographie geführten Ultraschalldämpfungsparameters nach dem vorhergehenden Anspruch, wobei die Sonde außerdem so konfiguriert ist, dass sie die kontinuierliche Vibration anwendet, wenn die Kontaktkraft zwischen der Sonde und dem viskoelastischen Medium größer als ein vorbestimmter Wert ist.

12. Sonde zur Messung der durch harmonische Elastographie geführten Ultraschalldämpfung nach Anspruch 10 oder 11, **dadurch gekennzeichnet, dass** der Ultraschallwandler vom Vibrator getragen wird.

13. Sonde zur Messung der durch harmonische Elastographie geführten Ultraschalldämpfung nach einem der Ansprüche 11 oder 12, die so konfiguriert ist, dass sie einen mittleren Ultraschalldämpfungsparameter berechnet, wobei der mittlere Ultraschalldämpfungsparameter aus einer Vielzahl von momentanen Ultraschalldämpfungsparametern und Qualitätskoeffizienten, die mit den momentanen Ultraschalldämpfungsparametern verbunden sind, berechnet wird.

14. Vorrichtung zur Messung der durch harmonische Elastographie geführten Ultraschalldämpfung, umfassend:

- Eine Sonde (PR) zur Messung der durch harmonische Elastographie geführten Ultraschalldämpfung nach Anspruch 10 oder 11 ;

- Eine Zentraleinheit (UC), die mit der Sonde (PR) verbunden ist und mindestens Rechenmittel für die Verarbeitung der reflektierten Ultraschallsignale, Anzeigemittel (SC) und Kontroll- und/oder Eingabemittel (ENT) umfasst.

## Claims

1. A method for measuring an ultrasound attenuation parameter guided by harmonic elastography comprising a step of applying, by means of a vibrator included in a probe in contact with a viscoelastic medium, a low-frequency continuous vibration, the low-frequency continuous vibration generating an elastic wave inside the viscoelastic medium and generating (CW), during propagation of the elastic wave, using an ultrasound transducer in contact with the viscoelastic medium, a series of ultrasound acquisitions, said series of ultrasound acquisitions comprising groups of ultrasound acquisitions, the groups of ultrasound acquisitions being generated with a repetition frequency (LPRF), each group of ultrasound acquisitions comprising at least one acquisition;

the ultrasound attenuation parameter being measured from the ultrasound acquisitions;
said method further comprising further a step of determining (CW_P), from the series of ultrasound acquisitions, at least one property of the propagation of the elastic wave inside the viscoelastic medium, and using the property of the propagation of the elastic wave inside the viscoelastic medium to calculate a real-time positioning indicator (RT_IP) of the probe with respect to the viscoelastic medium to be studied.

2. The method for measuring an ultrasound attenuation parameter guided by harmonic elastography according to the preceding claim, **characterised in that** it further comprises a step of displaying the real-time positioning indicator (RT_IP) in real time.

3. The method for measuring an ultrasound attenuation parameter guided by harmonic elastography according to one of claims 1 to 2, **characterised in that** the property of the propagation of the elastic wave measured is selected from the amplitude of the elastic wave, the phase of the elastic wave, the phase velocity of the elastic wave, the elasticity of the viscoelastic medium, the Young's modulus of the viscoelastic medium and the shear modulus of the viscoelastic medium.

4. The method for measuring an ultrasound attenuation parameter guided by harmonic elastography according to one of the preceding claims, **characterised in that** the step of applying a low-frequency continuous vibration is triggered only if the contact force between the vibrator and the viscoelastic medium is greater than a predetermined lower threshold.

5. The method for measuring an ultrasound attenuation parameter guided by harmonic elastography according to one of the preceding claims, **characterised in that** the step of applying a continuous low-frequency vibration is triggered only if the contact force between the vibrator and the viscoelastic medium is greater than a predetermined lower threshold and less than a predetermined upper threshold.

6. The method for measuring an ultrasound attenuation parameter guided by harmonic elastography according to one of the preceding claims, **characterised in that** the series of ultrasound acquisitions is formed by a repetition of groups comprising at least two ultrasound acquisitions having an intra-group repetition frequency (HPRF) of between 500 Hz and 10 kHz and a repetition frequency (LPRF) of between 10 Hz and 10 kHz.

7. The method for measuring an ultrasound attenuation parameter guided by harmonic elastography according to one of the preceding claims, **characterised in that** the repetition frequency (LPRF) is lower than the continuous vibration frequency (cSWF).

8. The method for measuring an ultrasound attenuation parameter guided by harmonic elastography according to one of the preceding claims, **characterised in that** the ultrasound attenuation parameter is an instantaneous parameter and a quality coefficient associated with the measurement of the instantaneous ultrasound attenuation parameter is calculated from a property of the propagation of the elastic wave inside the viscoelastic medium and/or from the properties of the ultrasound wave.

9. The method for measuring an ultrasound attenuation parameter guided by harmonic elastography according to the preceding claim, **characterised in that** an average ultrasound attenuation parameter is calculated from a plurality of instantaneous ultrasound attenuation parameters and quality coefficients associated with each instantaneous ultrasound attenuation parameter.

10. A probe for measuring an ultrasound attenuation parameter guided by harmonic elastography comprising:

   - a vibrator configured to apply a low frequency continuous vibration to the viscoelastic medium, the low frequency continuous vibration generating an elastic wave inside the viscoelastic medium;
   - an ultrasound transducer configured to emit a series of ultrasound acquisitions, said series of ultrasound acquisitions comprising groups of ultrasound acquisitions, the groups of ultrasound acquisitions being generated with a repetition frequency, each group of ultrasound acquisitions comprising at least one acquisition;
   - means for calculating and displaying in real time a probe positioning indicator, said positioning indicator being calculated based on a property of the propagation of the elastic wave, said property of propagation of the elastic wave being determined from the series of ultrasound acquisitions;

   the probe attenuation parameter being measured from the ultrasound acquisitions performed during the application of the low frequency continuous vibration.

11. The probe for measuring an ultrasound attenuation parameter guided by harmonic elastography according to the preceding claim, said probe being further configured to apply the continuous vibration when the contact force between the probe and the viscoelastic medium is greater than a predetermined value.

12. The probe for measuring ultrasound attenuation guided by harmonic elastography according to claim 10 or 11, **characterised in that** the transducer is carried by the vibrator.

13. The probe for measuring ultrasound attenuation guided by harmonic elastography according to one of claims 11 or 12, configured to calculate an average ultrasound attenuation parameter, said average ultrasound attenuation parameter being calculated from a multiplicity of instantaneous ultrasound attenuation parameters and quality coefficients associated with the instantaneous ultrasound attenuation parameters.

**14.** An ultrasound attenuation measurement device guided by harmonic elastography comprising:

- a probe (PR) for measuring ultrasound attenuation guided by harmonic elastography according to claim 10 or 11;
- a central processing unit (CPU) connected to the probe (PR) and comprising at least calculation means for processing the ultrasound signals reflected, display means (SC) and control and/or input means (ENT).

Figure 1

Figure 2

Figure 3

RT_Info

AJ
0 <> 1

Ph

(ms)

A

(µm)

UT

D = 0

Z

CW_Disp

T

ROI

Figure 4

Figure 5

PR
TOG
PT
A
VIB
I
TUS

Figure 6a

DEV
UC
SC
ENT
PR

Figure 6b

# EP 3 758 610 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 2739211 A1

### Littérature non-brevet citée dans la description

- **L. SANDRIN et al.** Transient Elastography : a new noninvasive method for assessment of hepatic fibrosis. *Ultrasound in Medecine and Biology,* 2003, vol. 29, 1705-1713
- **M. SASSO et al.** The controlled attenuation parameter (CAP): A novel tool for the non-invasive évaluation of steatosis using Fibroscan. *Clinical Research in Hepatology and Gastroenterology,* 2011
- **M. SASSO et al.** Controlled Atténuation Parameter (CAP): A Novel VCTE™ Guided Ultrasonic Atténuation Measurement for the Evaluation of Hepatic Steatosis: Preliminary Study and Validation in a Cohort of Patients with Chronic Liver Disease from Various Causes. *Ultrasound in Medecine and Biology,* 2010
- **M. SASSO et al.** Liver steatosis assessed by controlled attenuation parameter (cap) measured with the xl probe of the fibroscan: a pilot study assessing diagnostic accuracy. *Ultrasound in Medecine and Biology,* 2015